# EUROPEAN PATENT SPECIFICATION

(11) **EP 1 041 065 B1**
(45) Date of publication and mention of the grant of the patent: **20.08.2003**
(21) Application number: 00106443.5
(22) Date of filing: 24.03.2000
(51) Int. Cl.: C07C 273/16, A23C 9/142, B01D 61/02, B01D 61/14

(54) **Method for isolating urea using membranes and compositions thereof**
Verfahren zur isolierung von Harnstoff unter Verwendung von Membranen un deren Zusammensetzungen
Compositions et méthodes d'isolement de l'urée en utilisant des membranes

(30) Priority: 26.03.1999 JP 8272199; 26.03.1999 JP 8276899
(43) Date of publication of application: 04.10.2000
(73) Proprietor: SNOW BRAND MILK PRODUCTS, CO., LTD., Sapporo-shi Hokkaido 065-0043 (JP)
(72) Inventor: Minoru Morita, Toshima-ku, Tokyo (JP); Atsushi Serizawa, Kawagoe-shi, Saitama-ken (JP); Wataru Motsuchi, Belte Kawagoe No. 302, Kawagoe-shi, Saitama-ken 350-0042 (JP); Haruyoshi Yamamoto, Kawagoe-shi, Saitama-ken (JP); Masanori Kotani, Edogawa-ku, Tokyo (JP); Yoriko Yamane, Takamatsu-shi, Kagawa-Ken (JP)
(74) Representative: VOSSIUS & PARTNER

(56) References cited:
- EP-A- 0 044 872
- EP-A- 0 657 466
- WO-A-89/01468
- GB-A- 1 536 478
- CHEMICAL ABSTRACTS, vol. 115, no. 8, 26 August 1991 (1991-08-26) Columbus, Ohio, US; abstract no. 74159y, ZHOU, JIANHUI ET AL: "Several engineering equations in the design of reverse osmosis plants" page 144; column 1; XP002138713 & DESALINATION., vol. 80, no. 1, 1991, pages 15-30, ELSEVIER SCIENTIFIC PUBLISHING CO, AMSTERDAM., NL ISSN: 0011-9164
- CHEMICAL ABSTRACTS, vol. 70, no. 10, 10 March 1969 (1969-03-10) Columbus, Ohio, US; abstract no. 39274n, OHYA, HURUHIKO AND SOURIRAJAN, SRINIVASA: "Reverse-osmosis separation of urea in aqueous solutions using porous cellulose acetate membranes" page 115; column 2; XP002138714 & IND. ENG. CHEM., PROCESS DES. DEVELOP., vol. 8, no. 1, 1969, pages 131-142,
- CHEMICAL ABSTRACTS, vol. 95, no. 4, 27 July 1981 (1981-07-27) Columbus, Ohio, US; abstract no. 26215k, HUANG R Y M ET AL: "Investigations on nylon 4 membranes: synthesis and transport properties. II. Transport properties of nylon 4 polymer membranes" page 42; column 1; XP002138715 & JOURNAL OF APPLIED POLYMER SCIENCE., vol. 26, no. 6, 1981, pages 1907-1918, JOHN WILEY AND SONS INC. NEW YORK., US ISSN: 0021-8995
- CHEMICAL ABSTRACTS, vol. 91, no. 6, 6 August 1979 (1979-08-06) Columbus, Ohio, US; abstract no. 40544r, NAROLA B J ET AL: "Cellulose acetate (CA)-poly(methlymethacrylate) (PMMA) blend semipermeable membrane. Part IV. Application of CA-PMMA blend flat membrane for other than brackish water desalination" page 47; column 1; XP002138716 & INDIAN CHEM. J., vol. 13, no. 11, 1979, pages 23-24,
- CHEMICAL ABSTRACTS, vol. 91, no. 6, 6 August 1979 (1979-08-06) Columbus, Ohio, US; abstract no. 40545s, HINMANN P V ET AL: "Composite reverse-osmosis membranes prepared by plasma polymerisation of allylamine. Evaluation of emebrane performance for the treatment of wash water and its components" page 47; column 1; XP002138717 & JOURNAL OF APPLIED POLYMER SCIENCE., vol. 23, no. 12, 1979, pages 3651-3656, JOHN WILEY AND SONS INC. NEW YORK., US ISSN: 0021-8995
- CHEMICAL ABSTRACTS, vol. 114, no. 20, 20 May 1991 (1991-05-20) Columbus, Ohio, US; abstract no. 187069s, RAMACHANDHRAN V ET AL: "Poly(m-phenyleneisophthalamide) membranes for reverse-osmosis separations" page 70; column 2; XP002138718 & INT. J. POLYM. MATER., vol. 14, no. 3-4, 1990, pages 157-163,
- DATABASE WPI Week 198634, Derwent Publications Ltd., London, GB; AN 1986-223146 & JP 61 155 319 A (TEIKA SEIYASKU KK) 15 July 1986

## Description

The present invention relates to methods for readily isolating urea from a solution containing urea using membrane isolation technology. By using isolating methods of the present invention, urea can be readily isolated and recovered, for example, from natural products, and also urea can be readily isolated and removed from sewage.

Further, the present invention relates to compositions containing milk-derived urea and to methods for producing the same. The compositions containing milk-derived urea of the present invention can be readily isolated and recovered from milk or milk materials by using membrane isolating technology, and can be used as urea materials derived from natural products.

Isolation using membranes first started with the use of an RO membrane to convert seawater to freshwater. Until today, various membranes have been used in different fields. Use of membranes in a dairy industry started with the isolation of whey proteins from whey using an ultra filtration (UF) membrane. Today, all the membranes, i.e., an NF membrane, RO membrane, UF membrane, and microfiltration (MF) membrane are used.

Urea, a compound expressed by a chemical formula CO(NH₂)₂, was discovered by F.M. Rouelle in 1773, and the first organic compound that was successfully synthesized by F. Wohler in 1828. Since then, various methods of synthesizing urea have been proposed. Today, urea is synthesized by the direct method using ammonia and carbon dioxide as starting materials. Urea thus obtained is manufactured on a large scale, and used as an industrial raw material for products, such as urea resins, or as raw materials for chemical fertilizers, medicines and the like.

Urea is a final nitrogenous decomposition product of amino acids, nucleic acid bases and the like, and found in the blood and body fluids of vertebrates, and the urine of mammals. Urea is also found in nematodes, arthropods, mollusks, mushrooms, molds and the like. It is known that urea is present in the muscle of mollusks, such as sharks, in an amount of more than 1%, and also in the human blood in an amount of 0.02 to 0.03%. Urea is used as a raw material in agricultural chemicals, chemically synthesized products, medicines and the like. Furthermore, urea is admixed into hand creams or skin care creams to provide a moisturizing effect, or used as an effective component in diuretics.

Until now, no method of readily isolating and recovering urea from natural products has been reported. Thus, urea derived from natural products is not effectively used, and only synthesized urea is used.

On the other hand, the level of urea present in sewage is publicly regulated along with ammonia. The concentrations of ammonia and urea in sewage have to be less than 100 ppm. Accordingly, improved sewage treatment is needed. Proposed methods for removing urea today are hydrolyzation of urea into ammonia and carbon dioxide, and reaction of urea with nitrifying bacteria. However, these methods involve extremely complicated steps, such as heating at a high temperature or the use of microorganisms. Thus, it is currently difficult to satisfy these standards for sewage. Accordingly, development of methods of readily isolating and recovering urea from natural products, or readily isolating and removing urea from sewage is in great need.

Furthermore, there is a need to produce urea-containing compositions derived from natural products, which can be used in manufacturing products, such as cosmetics and medicines, where safety is a concern.

The present inventors intensively studied the development of methods of readily isolating and recovering urea from natural products with the view of effectively utilizing urea of natural origin. As a result, the present inventors found that urea actually permeates a nanofiltration (NF) membrane and reverse osmotic (RO) membrane, which were said to be permeable only to water and salts. Thus, the present inventors found that urea can be brought into the permeate side by treating a urea-containing fraction extracted from natural products or sewage containing urea, with an NF membrane or RO membrane.

This contrasts with the prior art, for example GB-A-1 536 478 which relates to a process for the separation of urea by relying on the rejection of urea by the osmotic membranes. Similarly, EP-A-0044872 concerns reverse osmotic membranes in which urea is rejected.

Furthermore, the present inventors found that a milk-derived composition containing urea at a level of more than 0.2% of the total solids can be obtained by treating natural products such as milk or milk materials with an NF membrane or RO membrane to bring a fraction containing urea into the permeate side. The present inventors then found that the milk-derived urea-containing composition can be used as a highly safe urea material of natural origin in manufacturing hand creams, diuretics and the like, and completed the present invention.

Accordingly, the present invention provides methods of readily isolating and recovering urea, which can be used as a raw material in cosmetics, medicines, fertilizers, and the like, from natural products, and methods of readily isolating and removing urea from sewage in the process of sewage treatment.

Furthermore, the present invention provides methods of producing a milk-derived urea-containing composition which can be used as a urea material of natural origin, by recovering the permeate by treating milk or milk materials with an NF membrane or RO membrane.

In the present invention, an NF membrane or RO membrane is used to isolate urea from solutions containing urea. Further, in the present invention, an NF membrane or RO membrane is used to produce compositions containing milk-derived urea. Unlike an ultrafiltration (UF) membrane or microfiltration (MF) membrane, an NF membrane or RO membrane used in the present invention has no micro pores on the membrane surface. Thus, the mechanism for the permeability of an NF membrane or RO membrane has not been revealed, and it is today difficult to theoretically explain why urea, which has been known not to permeate an NF membrane or RO membrane, can permeate these membranes. One theory is the intramembrane dissolution and dispersion theory that certain substances dissolve and disperse within a membrane while permeating. This intramembrane dissolution and dispersion theory may well explain why urea permeates an NF membrane or RO membrane. Namely, urea is considered to readily dissolve in an NF membrane or RO membrane.

Examples of RO membranes to be used in the present invention include Dasal-3 Desalination), HR-95 (Dow Danmark), and NTR-729HF (Nitto Denko). Examples of NF membranes include Desal-5 (Desalination), NF-45 (Dow Danmark), and NTR-7450(Nitto Denko). The shape of membranes to be used is not restricted, and can be selected depending on the solutions to be treated. If sanitary conditions are required, a spiral membrane is desirable. If solutions to be treated are highly viscous or contain large particles, a hollow fiber membrane is not suitable, and a spiral membrane is appropriately used.

A treatment temperature between 5C and 60C is desirable for treatment of solutions containing urea with an NF membrane or RO membrane. Urea could be decomposed at a temperature higher than 60C. Treatment efficiency decreases at a temperature below 5C. Other conditions such as the pressure and flow rate for the treatment can be the same as those ordinarily used in NF membrane or RO membrane treatment.

Thus, solutions containing urea can be treated with an NF membrane or RO membrane to bring the urea into the permeate side for isolation, then the isolated urea can be recovered or removed as required.

Further, in the present invention, milk or milk materials are treated with an NF membrane or RO membrane to bring urea-containing compositions into the permeate side, then these milk-derived urea-containing compositions are recovered, and, if necessary, concentrated or dried by ordinary methods. If the salt concentration of solutions containing urea is high, the salt concentration of the permeate also becomes high. Therefore, it is desirable to lower the salt concentration of the urea-containing solutions by desalting before the treatment, or by desalting the resultant urea-containing solutions. Such desalting can be done using ion-exchange resins or an electrodialysis (ED) membrane. Thus, urea-containing compositions, which are derived from natural products are highly safe, and which can be used for producing hand creams, diuretics and the like, can be readily isolated and recovered from milk or milk materials. The urea compositions thus isolated and recovered characteristically produce no ammonia or the like since they contain no urea decomposing enzyme, and make highly safe and readily usable urea materials.

Milk or milk materials can be treated with an NF membrane or RO membrane at a permeable solid concentration. However, it is better to treat at lower solid concentrations of milk or milk materials to obtain compositions having a high urea concentration, although the filtrating efficiency may decrease.

The following test examples will explain the permeation of urea through an NF membrane or RO membrane.

In the following test examples, quantitative measurements were carried out by the urease indole method for urea and by the ash method for minerals.

### Test Example 1

A solution containing 0.5% urea, 1% sodium chloride and 1% soybean proteins (model solution) was prepared. This model solution (5 kg) was subjected to a dialysis filtration with the addition of 5 volumes of water using an NF membrane having a salt suppression ratio of 50% (Desal-5, a product of Desalination). The membrane treatment was carried out at an operation temperature of 15C and at a pressure of 1.2 Mpa. The solution which permeated through this NF membrane into the permeate side was recovered, and concentrated with an evaporator. The resulting concentrate was desalted with cation exchange resins (IR-120B, a product of Organo), then anion exchange resins (IRA-410, a product of Organo).

The amount of urea contained in the resulting desalted concentrate was measured to be 24 g. It was revealed that urea can be isolated and recovered at a yield of 96% from the model solution, since 5kg of the model solution contained 25 g of urea.

The amount of minerals contained in the desalted concentrate was below the measurable limit.

### Test Example 2

A model solution containing 0.5% urea, 1% sodium chloride and 1% soybean proteins was prepared as described in Test Example 1. This model solution (5 kg) was subjected to a dialysis filtration with the addition of 5 volumes of water using an RO membrane having a salt suppression ratio of 95% (NTR-7450, a product of Nitto Denko). The membrane treatment was carried out at an operation temperature of 15C and at a pressure of 1.8 Mpa. The solution which permeated through this RO membrane into the permeate side was recovered, and concentrated with an evaporator.

The amount of urea contained in the resulting desalted concentrate was measured to be 19 g. It was revealed that urea can be isolated and recovered at a yield of 76% from the model solution, since 5kg of the model solution contained 25 g of urea.

The amount of minerals contained in the desalted concentrate was 3 g.

In the following examples, quantitative measurements were carried out by the urease indole method for urea and by the ash method for minerals, as described in the test examples.

### Example 1

Skim milk containing urea at a concentration of 11.6 mg/100 ml was concentrated 2 times using an RO membrane having a salt suppression ratio of 98% (Desal-3, a product of Desalination). The membrane treatment was carried out at an operation temperature of 50C and at a pressure of 1.8 Mpa. The solution which permeated through this RO membrane into the permeate side was isolated and recovered.

The urea concentration of the resulting solution was 10 mg/100 ml (urea recovery: 86.2%).

The mineral concentration of this solution was 12 mg/100 ml.

### Example 2

Sewage containing urea at a concentration of 1.1 mg/100 ml was subjected to a dialysis filtration with the addition of 3 volumes of water using an NF membrane having a salt suppression ratio of 50% (Desal-5, a product of Desalination). The membrane treatment was carried out at an operation temperature of 15C and at a pressure of 1.3 Mpa. The solution which permeated through this NF membrane into the permeate side was isolated, and recovered.

The urea concentration of the resulting solution was 0.98 mg/100 ml (urea recovery: 89.0%).

### Example 3

Skim milk (50 L) containing urea at a concentration of 11.0 mg/100 ml was concentrated 2.5 times using an NF membrane having a salt suppression ratio of 50% (Desal-5, a product of Desalination). The membrane treatment was carried out at an operation temperature of 50C and at a pressure of 1.3 Mpa. The resultant permeate isolated and recovered from this NF membrane treatment was freeze-dried to obtain 50 g of powder of a milk-derived urea-containing composition.

The composition of this powder of a milk-derived urea-containing composition is shown in Table 1. The urea concentration was 6.2%.

**Table 1**

| | |
|---|---|
| Water | 3.0(%) |
| Fat | 0.2 |
| Protein | 25.3 |
| Carbohydrate | 3.1 |
| Minerals | 68.4 |

### Example 4

Cheese whey containing urea at a concentration of 8.7 mg/100 ml was concentrated 2 times using an RO membrane having a salt suppression ratio of 98% (Desal-3, a product of Desalination). The membrane treatment was carried out at an operation temperature of 50C and at a pressure of 1.8 Mpa. The resultant permeate isolated and recovered from this RO membrane treatment was concentrated with an evaporator. The resulting concentrate was desalted with cation exchange resins (IR-120B, a product of Organo), then anion exchange resins (IRA-410, a product of Organo), and freeze-dried to obtain 5.5 g of powder of a mil-derived urea-containing composition.

The urea concentration of this powder of a milk-derived urea-containing composition was 81%.

Solutions containing urea, such as urea-containing natural products or fractions containing urea extracted from natural products, or sewage containing urea, can be treated with an NF membrane or RO membrane to isolate urea in the permeate side, then the isolated urea can be recovered, and if necessary, purified, concentrated or dried by ordinary methods. If the salt concentration of solutions containing urea is high, the salt concentration of the permeate also becomes high. Therefore, it is desirable to lower the salt concentration of the urea-containing solutions by desalting before the treatment, or by desalting the resultant urea-containing solutions. Such desalting can be done using ion-exchange resins or an electrodialysis (ED) membrane. Thus, urea which can be used as a raw material for chemically synthesized products, medicines, fertilizers and the like can be readily isolated and recovered from natural products. The urea compositions thus isolated and recovered characteristically produce no ammonia or the like since they contain no urea decomposing enzyme, such as urease, and are accordingly very useful. For the removal or urea, urea can be removed without further treatment. In the present invention, urea contained in sewage can be readily isolated and removed using an NF membrane or RO membrane.

Furthermore, according to the methods of isolating urea using an NF membrane or RO membrane of the present invention, urea can be readily isolated and recovered from urea-containing natural products or fractions containing urea extracted from natural products. Urea thus obtained can be used as a raw material for chemically synthesized products, medicines, fertilizers or the like.

Further, in the present invention, milk or milk materials are treated with an NF membrane or RO membrane, and the permeate is isolated and recovered to obtain milk-derived urea-containing compositions. The resulting milk-derived urea-containing compositions can be used as a urea material of natural origin for products which require safeness, such as hand creams and diuretics.

## Claims

1. A method of isolating urea comprising the steps of:
contacting a solution containing urea with a nanofiltration (NF) membrane or reverse osmotic (RO) membrane, said solution being selected from a natural product containing urea, a fraction containing urea extracted from a natural product, or sewage containing urea;
dissolving and dispersing the urea in the membrane at a temperature of 5-60°C under a pressure in a range including 1.3 MPa and 1.8 MPa;
bringing the urea into the permeate side; and
recovering the urea from the permeate.

2. A method as claimed in claim 1 wherein said solution containing urea is milk or a milk material.

3. A method of producing a urea-containing composition from milk or a milk material, comprising the steps of:
contacting milk or a milk material containing urea with a nanofiltration (NF) or reverse osmotic (RO) membrane;
dissolving and dispersing the urea in the membrane;
bringing the urea into the permeate side; and
recovering the permeate to obtain a composition containing milk-derived urea at a concentration of 0.2% or more of the total solid of the composition.

4. Use of a milk-derived urea-containing composition in the preparation of cosmetics or hand creams, said composition containing urea material of natural origin at a concentration of 0.2% or more of the total solid of the composition.

5. Use of a milk-derived urea-containing composition in the preparation of medicines, said composition containing milk-derived urea at a concentration of 0.2% or more of the total solid of the composition.

## Patentansprüche

1. Verfahren zum Isolieren von Harnstoff, umfassend die Schritte:
Inkontaktbringen einer harnstoffhaltigen Lösung mit einer Nanofiltrations(NF)membran oder einer Umkehrosmose(RO)membran, wobei die Lösung aus einem harnstoffhaltigen natürlichen Produkt, einer von einem natürlichen Produkt gewonnenen harnstoffhaltigen Fraktion oder harnstoffhaltigem Abwasser ausgewählt wird;
Lösen und Dispergieren des Harnstoffs in der Membran bei einer Temperatur von 5 - 60°C und bei einem Druck in einem Bereich, der 1,3 MPa bis 1,8 MPa einschließt;
Überführen des Harnstoffs auf die Permeatseite; und
Rückgewinnen des Harnstoffs aus dem Permeat.

2. Verfahren nach Anspruch 1, wobei die harnstoffhaltige Lösung Milch oder ein Milchmaterial ist.

3. Verfahren zum Herstellen einer harnstoffhaltigen Zusammensetzung aus Milch oder einem Milchmaterial, umfassend die Schritte:
Inkontaktbringen harnstoffhaltiger Milch oder des harnstoffhaltigen Milchmaterials mit einer Nanofiltrations(NF)membran oder einer Umkehrosmose(RO)membran;
Lösen und Dispergieren des Harnstoffs in der Membran;
Überführen des Harnstoffs auf die Permeatseite; und
Rückgewinnen des Permeats, um eine Zusammensetzung zu erhalten, die aus Milch gewonnenen Harnstoff in einer Konzentration von mindestens 0,2% des gesamten Feststoffgehalts der Zusammensetzung enthält.

4. Verwendung einer aus Milch gewonnnen, harnstoffhaltigen Zusammensetzung bei der Herstellung von Kosmetik oder Handcremes, wobei die Zusammensetzung Harnstoffmaterial natürlichen Ursprungs in einer Konzentration von mindestens 0,2% des gesamten Feststoffgehalts der Zusammensetzung aufweist.

5. Verwendung einer aus Milch gewonnnen, harnstoffhaltigen Zusammensetzung bei der Herstellung von Medikamenten, wobei die Zusammensetzung aus Milch gewonnenen Harnstoff in einer Konzentration von mindestens 0,2% des gesamten Feststoffgehalts der Zusammensetzung aufweist.

## Revendications

1. Méthode d'isolement de l'urée comprenant les étapes consistant à :
mettre en contact une solution contenant de l'urée avec une membrane de nanofiltration (NF) ou une membrane d'osmose inverse (OI), ladite solution étant choisie parmi un produit naturel contenant de l'urée, une fraction contenant de l'urée extraite d'un produit naturel ou une eau usée contenant de l'urée;
dissoudre et disperser l'urée dans la membrane à une température de 5 à 60°C sous une pression égale ou supérieure à 1,3 MPa et égale ou inférieure à 1,8 MPa ;
faire passer l'urée du côté perméat ; et
récupérer l'urée à partir du perméat.

2. Méthode selon la revendication 1. dans laquelle ladite solution contenant de l'urée est du lait ou une matière lactée.

3. Méthode de production d'une composition contenant de l'urée à partir de lait ou d'une matière lactée, comprenant les étapes consistant à :
mettre en contact du lait ou une matière lactée contenant de l'urée avec une membrane de nanofiltration (NF) ou une membrane d'osmose inverse (OI) ;
dissoudre et disperser l'urée dans la membrane ;
faire passer l'urée du côté perméat ; et
récupérer le perméat pour obtenir une composition contenant de l'urée dérivée du lait à une concentration égale ou supérieure à 0,2 % par rapport à la matière solide totale de la composition.

4. Utilisation d'une composition contenant de l'urée dérivée du lait dans la préparation de produits cosmétiques ou de crèmes pour les mains, ladite composition contenant une matière d'urée d'origine naturelle à une concentration égale ou supérieure à 0,2 % par rapport à la matière solide totale de la composition.

5. Utilisation d'une composition contenant de l'urée dérivée du lait dans la préparation de médicaments, ladite composition contenant de l'urée dérivée du lait à une concentration égale ou supérieure à 0,2 % par rapport à la matière solide totale de la composition.
